(19) Europäisches Patentamt
European Patent Office
Office européen des brevets

(11) EP 2 997 365 B1

(12) **EUROPÄISCHE PATENTSCHRIFT**

(45) Veröffentlichungstag und Bekanntmachung des Hinweises auf die Patenterteilung:
**23.05.2018  Patentblatt 2018/21**

(51) Int Cl.:
*G01N 33/49* *(2006.01)*      *G01N 21/49* *(2006.01)*
*G01N 15/14* *(2006.01)*

(21) Anmeldenummer: **14752580.2**

(22) Anmeldetag: **05.08.2014**

(86) Internationale Anmeldenummer:
**PCT/EP2014/066786**

(87) Internationale Veröffentlichungsnummer:
**WO 2015/024770 (26.02.2015 Gazette 2015/08)**

(54) **ANALYSEVERFAHREN ZUR KLASSIFIKATIONSUNTERSTÜTZUNG**

ANALYSIS METHOD FOR SUPPORTING CLASSIFICATION

PROCÉDÉ D'ANALYSE POUR L'AIDE À LA CLASSIFICATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.08.2013  DE 102013216362**

(43) Veröffentlichungstag der Anmeldung:
**23.03.2016  Patentblatt 2016/12**

(73) Patentinhaber: **Siemens Healthcare Diagnostics Products GmbH**
**35041 Marburg (DE)**

(72) Erfinder:
• **KAVSEK, Barbara**
  **A-8061 Sankt Radegund bei Graz (AT)**
• **LEDERER, Peter**
  **A-8044 Graz (AT)**
• **TAAL, Peter**
  **NL-4388 HK Oost-Souburg (NL)**
• **VAN DEN BOOGAART, Jan**
  **NL-5711 AG Someren (NL)**

(56) Entgegenhaltungen:
EP-A1- 2 280 278       WO-A1-2013/093913
US-A1- 2010 273 168     US-A1- 2011 178 716

EP 2 997 365 B1

**Beschreibung**

Die Erfindung betrifft ein Analyseverfahren zur Klassifikationsunterstützung, ein Ermittlungsverfahren zur Bestimmung von Parametern des Analyseverfahrens und ein Computerprogrammprodukt, sowie ein optisches Analysesystem.

Im medizinischen Bereich werden optische Analysesysteme und optische Verfahren verwendet, um organische Dispersionen, die zum einen aus einem Dispersionsmedium und zum anderen aus einer dispersen Phase bestehen, optisch zu analysieren. Die disperse Phase umfasst in der Regel Partikel, insbesondere organisches Material, wie zum Beispiel Zellen oder Zellenbestandteile. Unter dem Dispersionsmedium kann man sich Medien vorstellen, die die disperse Phase umfassen, verdünnen oder anderweitig aufnehmen, wie zum Beispiel Blutplasma.

Die Analyse der Bestandteile einer Dispersion kann mit optischen Methoden durchgeführt werden, wobei ein Lichtstrahl, insbesondere Laserstrahl, in die Dispersion fokussiert und anschließend auf verschiedene optische Merkmale hin untersucht wird. Auf diese Weise sind Messwerte zu bestimmten Merkmalen der Dispersion ermittelbar.

Aus US 2011/178716 A1, US 2010/273168 A1, EP 2 280 278 A1 und WO 2013/093913 A1 sind Analyseverfahren zur Klassifikationsunterstützung bekannt.

Aus WO 00/58727 ist ein Analysesystem bekannt, welches aus einer Lichtquelle besteht, deren Licht in eine Dispersionsumwälzungseinheit (Flowcell) fokussiert und anschließend ausgewertet wird. Die Auswertung bezieht sich insbesondere auf die spektrale Ermittlung der in der Dispersion absorbierten Wellenlängen des verwendeten Lichts. Zum anderen werden die Beugecharakteristiken im Nah- als auch im Fernfeld dokumentiert, um auf bestimmte Eigenschaften der Dispersion, insbesondere der in der Dispersion enthaltenen Partikel, wie zum Beispiel der roten Blutkörperchen, der weißen Blutkörperchen oder Blutplättchen, rückzuschließen. Eine optische, hämatologische Untersuchung, wie sie aus dem Stand der Technik bekannt ist, kann vorteilhafterweise automatisiert vorgenommen werden, wobei eine Reihe von optischen Merkmalen der Dispersion untersucht werden. Jedoch begrenzt die optische Analysemethode den Informationsgehalt, der zur Dispersion extrahierbar ist. Obwohl Informationen zum Absorbtionsverhalten, Häufigkeitswerten von weißen und roten Blutkörperchen oder Blutplättchen, gewonnen werden können, ist für die Durchführung vieler medizinscher Diagnosen der extrahierte Informationsgehalt immer noch unzulänglich.

Beispielsweise verhält es sich bei der Malaria-Diagnose so, dass als Dispersion eine Blutprobe des Patienten nicht automatisch untersucht werden kann, sondern auf händische Art und Weise unter dem Mikroskop im Labor untersucht werden muss. Die derzeit beste Methode (Goldstandard) für die Malariadiagnose ist der Blutausstrich auf einem Objektträger oder zwischen zwei Objektträgern. Malariaparasiten werden lichtmikroskopisch mittels Dünn- und Dickblutausstrichen nachgewiesen. Die Genauigkeit der Diagnose ist zu einem großen Anteil von der Qualität des Blutausstriches und der Erfahrung des Laborpersonals abhängig.

Charakteristisch für parasitär befallene rote Blutkörperchen ist eine besondere Ringform, die unter dem Mikroskop zu erkennen ist, jedoch ist eine sichere Diagnose nur dann gewährleistet, wenn eine ausreichende Anzahl dieser ringförmigen roten Blutkörperchen in der Dispersion der Blutprobe gezählt werden können. Aufgrund dieses Zählexperimentes kann ein Arzt erkennen, ob es sich um eine starke oder weniger starke Malariainfektion handelt. Leider ist das Zählen der ringförmigen roten Blutkörperchen unter dem Mikroskop sehr langwierig, aufwändig und fehlerbehaftet, sodass eine Malariadiagnose teuer und zu ungenau ist. Hinzu kommt, dass sich verschiedene Labore in verschiedenen Notationen auf unterschiedliche Bereiche hinsichtlich der gezählten ringförmigen roten Blutkörperchen beziehen. Auf diese Weise besteht zudem die Fragestellung, wie eine standardisierte, verlässliche Malariadiagnose oder auch Diagnosen anderer Krankheiten oder Negativzuständen zuverlässig durchgeführt werden können.

Der Erfindung liegt die Aufgabe zugrunde, ein optisches Analyseverfahren bereit zu stellen, welches den Informationsgehalt eines optischen Analyseverfahrens dahingehend verbessert, dass präzisere Aussagen zur Dispersion, insbesondere Diagnosen und Klassifizierungen, möglich sind.

Diese Aufgabe wird erfindungsgemäß gelöst durch ein Analyseverfahren zur Klassifikationsunterstützung gemäß Anspruch 1, einem Computerprogrammprodukt gemäß Anspruch 9 und einem optischen Analysesystem gemäß Anspruch 10.

Erfindungsgemäß beinhaltet das Analyseverfahren zur Klassifikationsunterstützung die Schritte:

- Ermittlung von Messwerten ($P_i$) zu optischen Merkmalen einer zu klassifizierenden Testdispersion, wobei die Testdispersion aus einem Dispersionsmedium, insbesondere Blutplasma, und einer dispersen Phase gebildet ist und die disperse Phase Zellen oder Zellenbestandteile, insbesondere organischen Materials, aufweist,
- Berechnung eines Klassifikationsindex Y, definiert durch:

$$Y = \sum_{i=1}^{n} l_i * (P_i - E_i)/\sigma_i,$$

wobei der Klassifikationsindex $Y$ von einer Anzahl $n$ der optischen Merkmale $i$ der Testdispersion, einem Signifikanzparameter $l_i$ des jeweiligen optischen Merkmals $i$, einem Mittelwert $E_i$ zum jeweiligen optischen Merkmal $i$ und einer Standardabweichung $\sigma_i$ zum jeweiligen optischen Merkmal $i$ abhängt.

Der Klassifikationsindex ist dafür vorgesehen, eine Auskunft zu geben, die es erlaubt zu einer Dispersion, insbesondere einer organischen Dispersion, eine Klassifizierung vorzunehmen. Die Klassifizierungen können dabei aus verschiedenen Bereichen der Medizin, Technik oder aus der Wahrscheinlichkeitstheorie oder aus ähnlichen Bereichen stammen. Mögliche Klassifikationen sind beispielsweise das Vorliegen oder das Nichtvorliegen einer Krankheit, genauso wie das Vorliegen oder das Nichtvorliegen einer Eigenschaft, die in einem allgemeinsten Sinne zu verstehen ist. Dabei ist die Anzahl der Klassen genauso wenig beschränkt, wie auch deren technisches Gebiet, so können zwei, drei oder mehrere Klassifikationsalternativen möglich sein und durch den Klassifikationsindex angezeigt werden.

Darüber hinaus ist die Klassifikation durch den Klassifikationsindex als Vorschlag anzusehen, der mit einer bestimmten Wahrscheinlichkeit zutrifft, jedoch nicht zwingend vorliegen muss. Somit ist es möglich, dass der Klassifikationsindex eine Klassifikation der Dispersion vorschlägt, dieser Vorschlag bezogen auf den Einzelfall mit einer gewissen, idealerweise geringen Wahrscheinlichkeit jedoch nicht zutrifft.

Unter einem Dispersionsmedium wird ein Medium, insbesondere ein flüssiges Medium, verstanden, welches in der Lage ist die disperse Phase derart aufzunehmen, dass die zur dispersen Phase gehörenden Zellen oder Zellenbestandteile durch eine Bewegung des Dispersionsmediums mittransportiert werden können. Beispielsweise ist es sinnvoll bei einer zu klassifizierenden Testdispersion, wie zum Beispiel einer Blutprobe, diese während der optischen Ermittlung von Messwerten zu den optischen Merkmalen in einem fließenden Zustand zu halten. Auf diese Weise ist es einerseits möglich eine für eine statistische Betrachtung erforderliche Durchmischung der Testdispersion sicherzustellen oder alternativ intrinsisch bedingte Änderungen der Dispersion, wie zum Beispiel einer Gerinnung bei Blutdispersionen, vorzubeugen.

Der Klassifikationsindex hängt von einer Anzahl der optischen Merkmale der Testdispersion ab. Ein Messwert ist einem optischen Merkmal der Testdispersion zuzuordnen, womit ein gewisser Informationsgehalt der Testdispersion durch die Messung des zugeordneten Messwertes entnommen werden kann. Ein optisches Merkmal kann beispielsweise eine Anzahl von bestimmten Zellen, beispielsweise rote Blutkörperchen, weiße Blutkörperchen oder dergleichen, innerhalb einer bestimmten Volumeneinheit sein. Es kann aber auch ein optisches Merkmal zur Form oder Absorptionscharakteristik der genannten Zellen sein. Diese Merkmalsliste ist nicht abschließend.

Die optischen Merkmale per se erlauben keine eindeutige Klassifizierung, Indizierung oder Diagnose, sodass ein einzelnes optisches Merkmal kaum verwertbare Informationen liefert. Jedoch tragen optische Merkmale in Bezug auf eine Klassifikation dennoch eine gewisse Signifikanz in sich. Um eine Unterstützung für eine Klassifikation zu erhalten, ist es vorteilhaft eine Anzahl bestimmter optischer Merkmale auszuwählen, die eine gewisse Signifikanz für diese Klassifikation enthalten können. In Abhängigkeit der jeweiligen Klassifikationsproblematik einer Ausführungsform, können technische Hintergründe oder technisches Vorwissen zur Auswahl der optischen Merkmale herangezogen werden, deren Messwerte dann dem Analyseverfahren zur Klassifikationsunterstützung zugrunde gelegt werden können. Diese zugrunde gelegten optischen Merkmale werden des Weiteren auch Merkmalssatz genannt. Beispielsweise im Falle einer Blutprobe einer Testdispersion, werden bei einer Malariadiagnoseunterstützung die optischen Merkmale herangezogen, die in Verbindung mit roten Blutkörperchen zustande kommen. Dies kann durch eine Vorkenntnis begründet werden, die auf die übliche mikroskopbasierte Diagnoseunterstützung zurückgeht, bei der ebenfalls rote Blutkörperchen untersucht werden. Jedoch können auch andere optische Merkmale, die sich auf andere Bestandteile der Testdispersion beziehen nach und nach zum Merkmalssatz hinzugenommen oder bereits dem Merkmalssatz angehörige Merkmale basierend auf der Signifikanz des Informationsgehaltes entfernt werden. So kann sich herausstellen, dass ein optisches Merkmal, obwohl es das rote Blutkörperchen betrifft, keine Aussagekraft im Hinblick auf Malaria besitzt und somit vom Merkmalssatz entfernt werden kann. Umgekehrt kann ein optisches Merkmal, welches beispielsweise eine Zelle betrifft, die mit dem roten Blutkörperchen wechselwirkt, und von daher ebenfalls bei einem parasitären Malariabefall eine Rolle spielt, über das entsprechende optische Merkmal dahingehend beitragen, dass dessen signifikanter Informationsgehalt für das Analyseverfahren zur Klassifikationsunterstützung bereitgestellt wird, indem das optische Merkmal für eine Malariadiagnoseunterstützung Berücksichtigung findet.

Ein Signifikanzparameter ist jeweils einem optischen Merkmal zugeordnet, wobei dieses durch dessen Betrag und/oder dessen Vorzeichen anzeigen kann, inwieweit sich der Messwert des jeweiligen optischen Merkmals auf den Klassifikationsindex auswirkt. Ferner kann anhand des Signifikanzparameters eine Rangfolge aufgestellt werden, anhand derer die Wichtigkeit des Merkmals für eine Klassifizierung, Diagnose oder dergleichen, hervorgeht. Anhand des Signifikanzparameters ist es auch möglich zu erkennen, ob der Messwert eines optischen Merkmals sinnvollerweise im Merkmalssatz für das Analyseverfahren zur Klassifikationsunterstützung weiter Verwendung finden sollte. Ein Ziel des Analyseverfahrens zur Klassifikationsunterstützung ist die möglichst genaue Bestimmung der Signifikanzparameter zu den als wichtig zu identifizierenden optischen Merkmalen.

Ferner hängt der Klassifikationsindex von Mittelwerten ab, wobei die einzelnen Mittelwerte jeweils optischen Merkmalen zuordenbar sind. Die Mittelwerte sind Mittelwerte von aus Eichdaten gewonnen Messwerten des jeweiligen optischen Merkmals. Der Klassifikationsindex hängt insbesondere von der Differenz des ermittelten Messwerts der Testdispersion und des Mittelwerts zum optischen Merkmal ab, womit der ermittelte Messwert umso stärker auf den Klassifikationsindex Einfluss nimmt, je weiter er von dem Mittelwert entfernt liegt.

Ferner hängt der Klassifikationsindex von der Standardabweichung des jeweiligen optischen Merkmals ab, womit eine Normierung erzielt wird, die eine natürliche Varianz des in Betracht zu ziehenden Messwerts des optischen Merkmals berücksichtigt wird, und somit die natürliche Varianz des Messwertes keinen Einfluss auf den Klassifikationsindex nehmen kann, wobei gleichzeitig kein Ungleichgewicht zwischen den Summanden der Summe in der Klassifikationsindexformel entsteht. Gemäß dem Analyseverfahren zur Klassifikationsunterstützung ist der Mittelwert des optischen Merkmals und die Standardabweichung des Messwerts des optischen Merkmals auf der Basis von ersten Eichdaten ermittelt worden, wobei die ersten Eichdaten von Dispersionen mit einer negativen Klassifikation abgeleitet sind, insbesondere von den ersten und zweiten Eichdaten abgeleitet sind, wobei die zweiten Eichdaten von Dispersionen mit positiver Klassifikation abgeleitet sind. Bei dieser Ausführungsform sind lediglich zwei Klassen zur Klassifizierung vorgesehen. Beispielsweise ist es bei einem Analyseverfahren zur Klassifikationsunterstützung bei Krankheitsdiagnosen schlichtweg erforderlich, dass eine Aussage, ob die Testdispersion von einem kranken Patienten stammt oder von einem gesunden Patienten. Daher werden die Mittelwerte von Messwerten eines bestimmten optischen Merkmals im Hinblick darauf mit dem Vorwissen betrachtet, ob die Testdispersion einer positiven Klassifikation oder einer negativen Klassifikation zuzuordnen ist. Beispielsweise kann der Mittelwert mit Messwerten des optischen Merkmals gebildet werden, bei denen eine negative Klassifikation bekannt ist. Dies würde bei der Krankheitsdiagnose gewissermaßen eine Eichung mit einem Mittelwert von gesunden Patienten entsprechen. Damit wird die Abweichung des gemessenen Messwertes der Testdispersion von diesem Mittelwert als der Informationsgehalt gewertet, der im Hinblick auf die Klassifikation Relevanz hat. Liegt der ermittelte Messwert der Testdispersion sehr nahe beim Mittelwert oder ist sogar mit diesem identisch, so liefert das zugehörige optische Merkmal keinen Beitrag.

Die ersten Eichdaten können von Dispersionen negativer Klassifikation abgeleitet sein, aber auch alternativ von Dispersionen mit einer positiven Klassifikation. Somit sind die zweiten Eichdaten jeweils der anderen Klassifikation zugehörig als die der ersten Eichdaten.

Grundsätzlich werden Eichdaten aus an Dispersionen gemessenen Messwerten zu optischen Merkmalen ermittelt, die idealerweise bei einer großen Vielzahl von Dispersionen festgestellt wurde, um eine statistische Aussagekraft zu gewährleisten, die für eine optimale Klassifikationsunterstützung erforderlich ist. Diese Eichdaten können mittels Klassifikationsverfahren des Stands der Technik ermittelt worden sein, wie zum Beispiel im Falle der Malariadiagnose mittels Blutausstriche und deren mikroskopischer Beurteilung. Zu den Eichdaten sollten ferner ausreichend viele Dispersionen unterschiedlicher Klassifikationen zugrunde liegen, idealerweise im Wesentlichen gleich viele Datensätze zur jeweiligen Klassifikation. Die Signifikanzparameter sind von den ersten und von den zweiten Eichdaten mittels einer Diskriminanzanalyse abgeleitet, wobei bei der Diskriminanzanalyse insbesondere eine bayes'sche Theorie verwendet wird.

Bei der Diskriminanzanalyse wird der Klassifikationsindex als Zufallsvariable interpretiert, wobei eine Wahrscheinlichkeit des Vorliegens einer Klassifikation über die Häufigkeit des Klassifikationsindex aufgetragen werden kann. Auf diese Weise liefert der Klassifikationsindex eine Wahrscheinlichkeitsverteilung, die sowohl Testdispersionen mit positiver als auch negativer Klassifikation beinhaltet, oder allgemein, mit allen vorgesehenen Klassifikationen. Die Diskriminanzanalyse beschreibt nun, wie es anhand der Wahrscheinlichkeitsverteilung über den Klassifikationsindex Entscheidungen zu treffen sind, die zu einer treffsicheren Klassifizierung führen.

Bei einer besonderen Ausführungsform, kann eine Klassifikationsregel angegeben werden, aufgrund derer Wertebereiche des Klassifikationsindex einer bestimmten Klassifikation zugeordnet werden können. Im Spezialfall der Zweiklassen-Klassifikation (positiv/negativ, gut/schlecht, minus/plus) kann auch ein Schwellenwert für den Klassifikationsindex anzugeben sein, der den Wertebereich des Klassifikationsindex in zwei Intervalle aufteilt und somit ebenfalls zu einer Klassifikationssregel führt. Beim Ausführungsbeispiel der Krankheitsdiagnose, kann bei Unterschreitung des angegebenen Schwellenwerts eine negative Diagnose vorgeschlagen und bei Überschreitung der Schwelle eine positive Diagnose vorgeschlagen werden.

Beispielsweise kann die bayes'sche Theorie eingesetzt werden, wobei priore und posteriore Wahrscheinlichkeit in die Berechnung mit einbezogen werden. Dabei muss genügend Wissen beziehungsweise Eichdaten, von verlässlichen priore Wahrscheinlichkeiten vorliegen, damit die Theorie zum Tragen kommt. Hierbei werden als priore Wahrscheinlichkeiten, Wahrscheinlichkeiten angesehen, die mit Vorwissen geschätzt sind und posteriore Wahrscheinlichkeiten als Wahrscheinlichkeiten die nachträglich errechnet wurden. Hierbei ist die Entfernung der Schwerpunkte zu den Testdaten zu berücksichtigen.

Bei einer vorteilhaften Ausführungsform ist der Mittelwert des Messwerts eines optischen Merkmals das arithmetische Mittel mehrerer Messwerte von ersten und/oder zweiten Eichdaten. Alternativ kann der Mittelwert eines Messwerts zu einem optischen Merkmal auch ein Schwerpunkt einer Gruppe von Punkten im ein- oder mehrdimensionalen Raum sein. Erfindungsgemäß ist basierend auf einem mittleren Klassifikationsindex, insbesondere einem ersten Schwerpunkt, der ersten Eichdaten und einem mittleren Klassifikationsindex, insbesondere einem zweiten Schwerpunkt, der zweiten Eichdaten ein Schwellenwert zur Klassifikationsunterstützung verwendbar, wobei eine positive Klassifikation vorliegt, wenn der Klassifikationsindex größer ist als der Schwellenwert und negative Klassifikation vorliegt, wenn der Klassifikationsindex kleiner als der Schwellenwert ist. Der Schwellenwert kann in Abhängigkeit der mittleren Klassifikationsindizes gewählt werden, wobei der Schwellenwert beispielsweise das arithmetische Mittel der beiden mittleren Klassifikationsindizes sein kann. Jedoch kann der Schwellenwert ebenfalls auf die Varianz von Klassifikationsindizes zurückgehen, die in Abhängigkeit der Klassifikation stärker oder weniger variieren können. Grundsätzlich kann die Ermittlung des Schwellenwertes auf Basis der Diskriminanzanalyse stattfinden. Beispielsweise kann der Schwellenwert derart zwischen den beiden mittleren Klassifikationsindizes festgelegt werden, dass positive und negative Klassifikation am Schwellenwert gleichermaßen wahrscheinlich ist. Erfindungsgemäß zeigt eine positive Klassifikation ein Vorliegen eines Mangels, eines parasitären Befalls oder eines unnormalen Zustandes an, wobei der Mangel, insbesondere eine Anämie, insbesondere eine Mittelmeer Anämie oder eine Sichelzellanämie, ist und wobei der parasitäre Befall, insbesondere eine Leishmaniose oder eine andere parasitäre Infektion ist. Das genannte Vorliegen eines Mangels, eines parasitären Befalls oder eines unnormalen Zustandes kann stets der Testdispersion zugeordnet werden, sodass eine Aussage oder Diagnose zur Testdispersion möglich ist. Zur Berechnung des Klassifikationsindex werden wenigstens zwei optische Merkmale berücksichtigt:

$$Y\ (n\ =\ 2)\ =\ l_1 * (P_1\ -\ E_1)/\sigma_1\ +\ l_2 * (P_2\ -\ E_2)/\sigma_2,$$

wobei n = 2 und ein erstes optisches Merkmal ein Maß für eine optische Streuung in eine transversale X-Richtung zu einem Analysestrahl ist und ein zweites optisches Merkmal ein Maß für eine optische Streuung in einer zur X-Richtung senkrechten und zum Analysestrahl ebenfalls senkrechten Y-Richtung ist, wobei der Analysestrahl durch die Testdispersion verläuft. Bei einer höheren Anzahl n erhöht sich auch die Anzahl der Summanden des Klassifikationsindex Y entsprechend. Bei der Wahl der Anzahl kann stets zwischen Rechenaufwand und zusätzlichem Informationsgehalt abgewogen werden.

In Abhängigkeit des Einzelfalls, kann der Merkmalssatz sehr klein gewählt werden, das heißt, dass nur wenige, idealerweise zwei, optische Merkmale ausreichen, um eine Klassifikation sicher genug vorzuschlagen. Die Messwerte der optischen Merkmale, die zur Berechnung des Klassifikationsindex verwendet werden, sind für die Klassifikation vorteilhafterweise auch relevant, wobei der Klassifikationsindex aus einer Summe gebildet wird, deren Anzahl der Summanden der Anzahl der verwendeten, optischen Merkmale entspricht.

Vorteilhafterweise verläuft der Analysestrahl im Wesentlichen in einer Z-Richtung, die sowohl auf der X-, als auch auf der Y-Richtung senkrecht steht. In der Z-Richtung kann der Analysestrahl beispielsweise durch optische Linsen, optische Filter oder dergleichen fokussiert, defokussiert oder anderweitig konditioniert worden sein, um in der Testdispersion einen optimalen Strahldurchmesser aufzuweisen. Nach der Transmission durch die Testdispersion wird der Analysestrahl ausgewertet, wobei beispielsweise Nahfeldstreuung, Fernfeldstreuung und/oder Wellenlängenabsorptionen im Sinne einer Spektralanalyse berücksichtigt werden. In einer Ausführungsform umfasst das Analyseverfahren ferner ein Ermittlungsverfahren zur Bestimmung von Analyseparametern des erfindungsgemäßen Analyseverfahrens, wobei für eine festgelegte Anzahl von optischen Merkmalen die Analyseparameter der Standardabweichung, des Mittelwerts und des Signifikanzparameter, basierend auf den ersten und/oder den zweiten Eichdaten ermittelt werden. Dabei ist es nicht immer erforderlich, dass alle Typen von Analyseparametern einer Anpassung oder Ermittlung unterzogen werden. Beispielsweise ist es denkbar, dass die Mittelwerte, beispielsweise als Schwerpunkte, aus von aus Eichdaten gewonnenen Messwerten der jeweiligen optischen Merkmale gebildet werden. Entsprechendes gilt für die zugehörigen Standardabweichungen der aus den ersten und/oder den zweiten Eichdaten gewonnenen Messwerte.

Zumindest die Signifikanzparameter müssen beim Ermittlungsverfahren zur Optimierung der Klassifikationsunterstützung variiert werden, um eine Gewichtung der Summanden des Klassifikationsindex dahingehend festzulegen, dass eine Klassifikation möglichst eindeutig ist. Dies ist genau dann der Fall, wenn Wertebereiche des Klassifikationsindex, die jeweils einer Klassifikation zuzuordnen sind, sich untereinander nicht überlappen. Eine Nichtüberlappung wird in der Praxis äußerst selten vorkommen, jedoch ist darauf hin zu optimieren, dass die genannten Wertebereiche möglichst wenig überlappen.

Vorteilhafterweise wird wenigstens ein Kontrollparameter, insbesondere ein Maß für die Überlappung der vorgennannten Wertebereiche, basierend auf den Analyseparametern, insbesondere basierend auf den Signifikanzparametern, zur Bewertung der Klassifikationsunterstützung berechnet. Die Klassifikationsunterstützung wird umso besser, je mehr die Wertebereiche unterschiedlicher Klassifikationen voneinander separiert sind, beziehungsweise je weniger diese überlappen.

Vorteilhafterweise wird wenigstens ein Teil der Analyseparameter nach einer Bewertung durch einen ersten Kontrollparameter angepasst, wobei insbesondere eine Anpassung des wenigstens einen Analyseparameters und die Bewertung der Klassifikationsunterstützung solange abwechselnd ausführbar ist, bis eine Verbesserung nicht mehr möglich oder nicht mehr wünschenswert ist. Die vorliegenden ersten und zweiten Eichdaten führen bei der Variation der Analyseparameter oder eines Teils der Analyseparameter stets zu einer unterschiedlichen Wahrscheinlichkeitsverteilung des Klassifikationsindex der genannten Eichdaten. Idealerweise sollen erste und zweite Eichdaten nachweislich zu separierbaren Wahrscheinlichkeitsdichteverteilungen unterschiedlicher Klassifikationen führen. Der erste Kontrollparameter könnte beispielsweise ein Minimum zwischen den genannten Verteilungen sein, wobei der erste Kontrollparameter bei der fortwährenden Anpassung der Analyseparameter oder deren Teil möglichst immer geringer ausfällt, sodass eine Separation der beiden Verteilungen umso deutlicher herausgestellt wird. Es können auch andere erste Kontrollparameter gewählt werden, die beispielsweise durch separate Kurvenfits der genannten Wahrscheinlichkeitsverteilungen ermittelt werden. Dabei können auch mehrere erste Kontrollparameter eine Rolle spielen, wobei diese aus den Fitparametern gewonnen werden, oder schlicht mit einem oder mehreren der Fitparameter identisch sind.

Vorteilhafterweise sind/ist der wenigstens eine Teil der Analyseparameter die Signifikanzparameter und/oder der Schwellenwert. Grundsätzlich muss der anzupassende Teil der Analyseparameter nicht mit einem Typ von Analyseparameter, wie zum Beispiel Signifikanzparameter, Standardabweichung oder Mittelwert, identisch sein, sondern kann auch Parameter unterschiedlichen Typs beinhalten. Auch der Schwellenwert kann an die Wahrscheinlichkeitsverteilung des Klassifikationsindex angepasst werden, falls sich für die Verteilungen unterschiedlicher Klassifikation Verteilungsfunktionen abzeichnen, die sich auf den Schwellenwert auswirken können.

Bei einer vorteilhaften Ausführungsform ist das Ermittlungsverfahren oder das Analyseverfahren mit einer reduzierten Anzahl von optischen Merkmalen erneut ausführbar, sobald eine Verbesserung unter Berücksichtigung des ersten Kontrollparameters nicht mehr möglich, oder nicht mehr erwünscht ist, wobei die Analyseparameter eines nicht signifikanten, optischen Merkmals oder mehrerer nicht signifikanter, optischer Merkmale nicht mehr berücksichtigt werden, wobei eine fehlende Signifikanz eines optischen Merkmals anhand des jeweiligen Signifikanzparameters festgestellt wird. Da im Vorhinein meist nicht bekannt ist, welche Messwerte von optischen Merkmalen zur Aussagekraft des Klassifikationsindex tatsächlich beitragen können, können beispielsweise eine große Anzahl Messwerte optischer Merkmale anfangs berücksichtigt werden - idealerweise alle verfügbaren Messwerte, wobei durch das Ermittlungsverfahren nach und nach Messwerte optischer Merkmale ausgeschlossen werden können, weil diese keinen oder nur einen sehr geringen Beitrag zur Aussagekraft des Klassifikationsindex aufweisen. Ausschlaggebend hierbei ist der Signifikanzparameter, der dann auf eine Signifikanz hindeutet, wenn er deutlich ungleich Null ist.

Signifikanzparameter, die sehr klein sind und/oder nahezu Null, können gegebenenfalls zum Ausschluss des Messwerts des jeweiligen optischen Merkmals führen. Auf diese Weise wird zum einen die Aussagekraft der Klassifikationsunterstützung verbessert, zum anderen werden aufgrund der Nichtberücksichtigung der Rechenaufwand eines Computers, auf den beispielsweise das Ermittlungsverfahren als Computerprogramm implementiert ist, weniger Rechenleistung aufwenden müssen.

Bevorzugt liegt eine fehlende Signifikanz vor, wenn der Einfluss des optischen Merkmals auf den Klassifikationsindex gering ist oder nicht vorliegt. Dennoch kann auf Messwerte verschiedener optischer Merkmale verzichtet werden, wenn Messwerte anderer optischer Merkmale genug Informationsgehalt besitzen. In diesem Fall ist es denkbar, beispielsweise mit einem Merkmalssatz zu beginnen, der vergleichsweise klein ist und sukzessiv weitere Messwerte anderer optischer Merkmale zum Merkmalssatz hinzuzufügen, womit im Einzelfall getestet werden kann, ob eine Verbesserung der Klassifikationsunterstützung erzielt worden ist.

Gegenstand der Erfindung ist des Weiteren ein Computerprogrammprodukt gemäß Anspruch 9 Dazu ist es vorteilhaft, wenn die ersten und/oder zweiten Eichdaten den Computer in geeigneter Weise als Datensätze vorliegen, damit eine Erfindung der Analyseparameter auf computerimplementierter Basis herbeigeführt werden kann. Vorteilhaft ist insbesondere, wenn Vergleichsdaten existieren, die nicht zum Eichen herangezogen wurden, aber anhand derer das Analyseverfahren auf seine Klassifikationsunterstützung getestet werden kann.

Vorteilhafterweise kann das Computerprogrammprodukt, insbesondere Compact Discs oder dergleichen, einen installierbaren Algorithmus enthalten. Der Algorithmus führt Programmschleifen mit entsprechender Abbruchsbedingung aus, die sowohl für die Ermittlung von Analyseparametern herangezogen werden können, aber auch zur Eliminierung von Analyseparametern verwendbar sein können.

Gegenstand der Erfindung ist auch ein optisches Analysesystem, insbesondere ein hämatologisches Analysesystem, gemäß Anspruch 10. Derartige Systeme stellen beispielsweise hämatologische Analysegeräte dar, die automatisiert eine ganze Reihe verschiedener Messwerte ermitteln, die optischen Merkmalen zuordenbar sind. Die Anzahl automatisiert ermittelter Messwerte kann von mehreren Hundert bis in die Tausende, beispielsweise eintausend oder zweitausend, gehen.

Weitere vorteilhafte Ausbildungen und bevorzugte Weiterbildungen der Erfindung sind der Figurenbeschreibung und/oder den Unteransprüchen zu entnehmen.

Im Folgenden wird die Erfindung anhand der in den Figuren dargestellten Ausführungsbeispiele näher beschrieben und erläutert. Es zeigen:

FIG 1  Ein optisches Analysesystem gemäß dem Stand der Technik,

FIG 2  eine schematische Darstellung eines Teils des Datenflusses gemäß dem erfindungsgemäßen Analyseverfahren,

FIG 3  eine Darstellung von Signifikanzparametern zum zugehörigen Messwert eines jeweiligen Merkmals, geordnet nach deren Betragsgröße,

FIG 4  ein Schema zur Ermittlung von Analyseparametern und ein Qualitätstest zum Analyseverfahren,

FIG 5  eine schematische Verdeutlichung von Programmschleifen zur Verbesserung der Klassifikationsunterstützung,

FIG 6  zwei grafische Darstellungen zur Verwendung in der Diskriminanzanalyse,

FIG 7  eine zweidimensionale grafische Darstellung zur Kontrolle und Verbesserung der Klassifikationsunterstützung,

FIG 8  eine grafische Darstellung eines Spezifitätshistogramms, und

FIG 9  eine Darstellung eines Sensitivitätshistogramms.

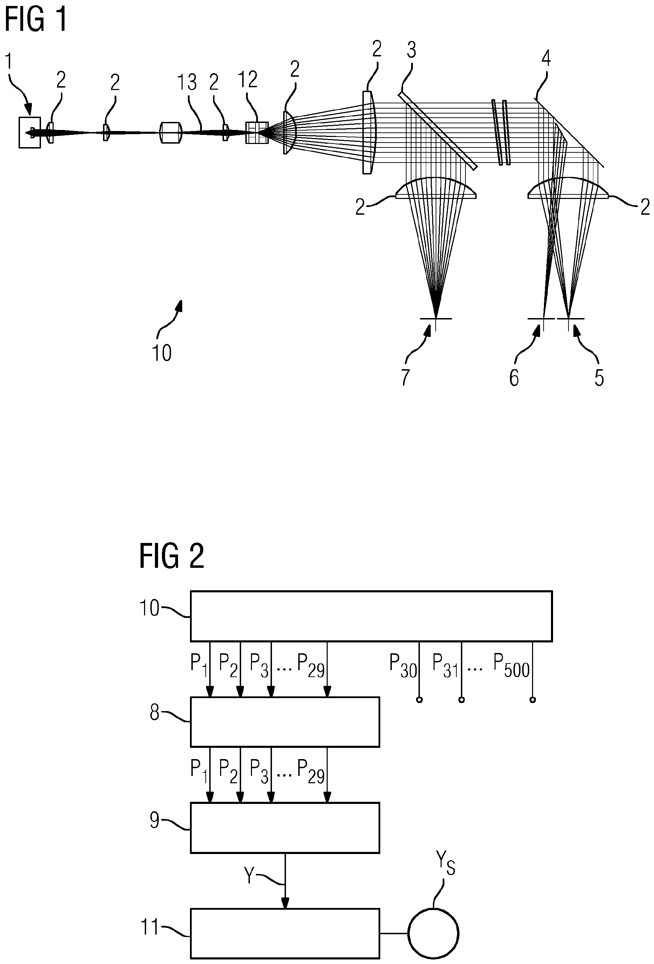

FIG 1 zeigt ein optisches Analysesystem 10, welches mit einer Lichtquelle 1, wie zum Beispiel einem Laser, einen Analysestrahl 13 generiert, der in verschiedenen Linsen 2 derart fokussiert wird, das der Analysestrahl 13 in einer organischen Dispersion 12 einen Fokus aufweist, wobei anschließend durch weitere Linsen 2 eine weitere Fokussierung des Strahles vorgenommen wird, sodass dieser großflächig durch einen Strahlteiler 3 zum einen in einem spektralen Sensor 7, wie zum Beispiel einem Spektrometer, analysierbar ist und auch über den Spiegel 4 Fernfeldbeugungsmuster 5 und Nahfeldbeugungsmuster 6 in jeweils einem Sensor detektierbar sind. Auf diese Weise lassen sich eine ganze Reihe von Messwerten, die einem optischen Merkmal der organischen Dispersion 12 zuzuordnen ist auf automatisierte Art und Weise bestimmen.

FIG 2 zeigt das optische Analysesystem 10 mit der Ausgabe von Messwerten $P_i$, wobei i von i = 1 bis 500 laufen kann und zur Durchnummerierung der optischen Merkmale verwendet wird. In diesem Ausführungsbeispiel werden die Messwerte $P_1$ bis $P_{29}$ aus dem optischen Analyseverfahren in einer nachgeschalteten Datenverarbeitung 8, die beispielsweise in einem Computer vorgenommen werden kann, aufbereitet, sodass die Messwerte $P_1$ in einer multivariaten Analyse einsetzbar sind. Die multivariate Analyse besteht darin ein Ausführungsbeispiel eines erfindungsgemäßen Analyseverfahrens auszuführen und einen Klassifikationsindex Y zu bestimmen, der der organischen Dispersion 12 aus FIG 1 zugeordnet werden kann.

Der Klassifikationsindex Y kann dabei beispielsweise zusammen mit einem Schwellenwert $Y_s$ ausgegeben werden, sodass für den Betreiber des optischen Analysesystems 10 ersichtlich ist, wie eine Klassifikation vorgenommen werden kann. Dabei handelt es sich jedoch nur um eine Klassifikationsunterstützung, bei der ein Klassifikationsvorschlag vom Analyseverfahren ausgegeben wird. Dies bedeutet, dass zwar ein Klassifikationsvorschlag durch das System gegebenenfalls feststeht, nämlich dann wenn der Klassifikationsindex Y über oder unter einer Schwelle $Y_s$ liegt, aber wenn beispielsweise eine Diagnose einer Krankheit vorgenommen werden soll, obliegt es immer noch dem Arzt des Patienten, der die organische Dispersion in Form einer Blutprobe zur Verfügung gestellt hat, die Diagnose einer vorliegenden Krankheit zu stellen oder nicht. Liegt der Klassifikationsindex Y genau auf dem Schwellenwert $Y = Y_s$ liefert das optische Analysesystem keine Klassifikationsunterstützung.

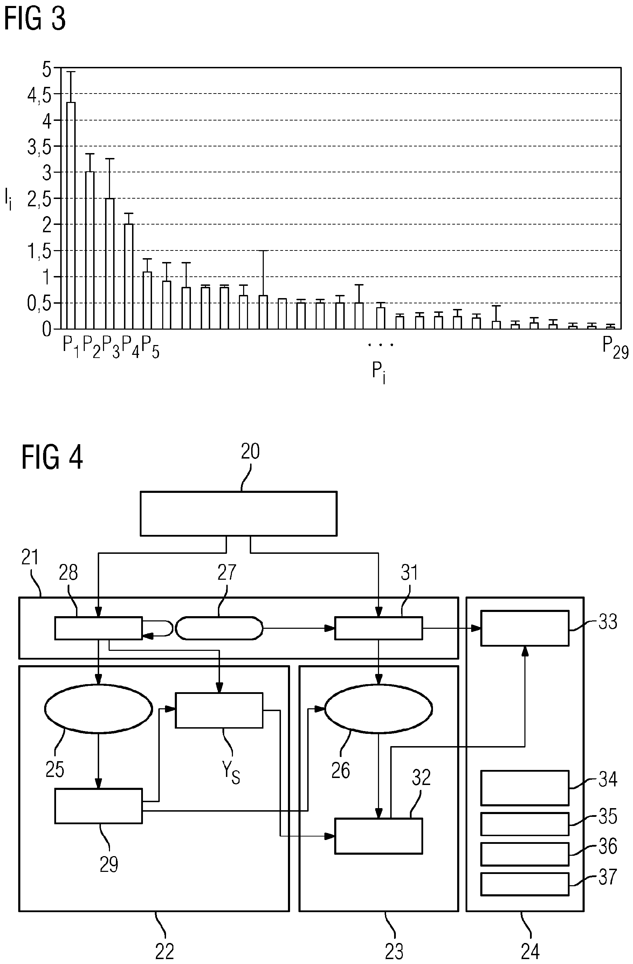

FIG 3 zeigt ein Diagramm mit Signifikanzparametern $I_i$, wobei die Signifikanzparameter $I_i$ über dem jeweiligen Messwert $P_i$ abgetragen worden sind. Dabei erhielten die Messwerte $P_i$ in Abhängigkeit vom Betrag des zugehörigen Signifikanzparameters $I_i$ die laufende Nummer i. Mit anderen Worten, die Reihenfolge, die durch den Zähler i gegeben ist, gibt auch gleichzeitig Auskunft über den Informationsbeitrag des Messwerts zum Klassifikationsindex wieder.

Das gezeigte Diagramm der FIG 3 bezieht sich auf die gleichen Messwerte $P_i$, die auch in FIG 2 gezeigt werden.

FIG 4 zeigt am Beispiel einer Klassifikationsunterstützung für die Malariadiagnose, bei der die Analyseparameter gefunden werden sollen, indem die Qualität der Klassifikationsunterstützung überprüft wird.

Die Malariadiagnoseunterstützung 20 basiert auf einer Trainingsphase 28 und einer anschließenden Testphase 31. Hierbei ermöglicht die Testphase 31 die Standardisierung 27 der Trainingsphase 28.

Zunächst wird das Training 22 anhand einer Wertebelegung 25 für die Analyseparameter $Y_s$, $E_i$, $I_i$, $\sigma_i$ begonnen, wobei anhand von Eichdaten eine Wahrscheinlichkeitsverteilung des Klassifikationsindex generiert wird und anhand dieser Wahrscheinlichkeitsverteilung eine Skalierung vorgenommen wird, die es ermöglicht eine Schwelle $Y_s$ anzugeben. War die Skalierung 29 erfolgreich, so wird die Wertebelegung der Analyseparameter Ys, $E_i$, $I_i$, $\sigma_i$ festgehalten, um im Analyseverfahren verwendet zu werden.

In einer zweiten Phase, der Vorhersage 23, findet ein Test anhand von Testdaten statt, die vorzugsweise nicht zu den Eichdaten gehören, sondern aus einem anderen Datensatz oder einer anderen Untersuchung oder dergleichen entnommen worden sind.

Aufgrund des gefundenen Satzes von Analyseparametern Ys, $E_i$, $I_i$, $\sigma_i$, die insbesondere die Signifikanzparameter $I_i$ einschließen, werden Klassifikationen von Testdispersionen vorgenommen, zu denen aufgrund der Eichdaten, bereits eindeutige Klassifikationen vorliegen.

Die Klassifikation 32 ist die Klassifikation, die dem Analyseverfahren zugeordnet werden kann.

Im Ausführungsbeispiel werden die Sensitivität und Spezifität betrachtet, um die Klassifikationsgenauigkeit, beziehungsweise bei einer Malaria Klassifikationsunterstützung, die Diagnosegenauigkeit festzustellen.

Die Spezifizität wird definiert als Wahrscheinlichkeit einer negativen Diagnose, gesetzt den Fall, dass diese Klassifikation richtig ist.

Sensitivität wird als Wahrscheinlichkeit einer positiven Klassifikation definiert, gesetzt den Fall, dass diese Klassifizierung korrekt ist.

Die Korrektheit wird bei Sensitivität, als auch bei der Spezifizität mit den Eichdaten in einem Vergleich festgestellt.

Zusätzlich sind auch andere Kriterien zu berücksichtigen, die gegebenenfalls alternativ oder optional Verwendung finden können, nämlich eine kanonische Korrelation 34, wilk's Lambda 35, Fit-Parameter $Chi^2$ 36 oder eine P-Wert 37 Betrachtung.

Nach der Qualitätskontrolle 24 kann entschieden werden, ob das Analyseverfahren eine effiziente Klassifizierungsregel bieten kann, die den Qualitätsstandards medizinischer Analysesysteme genügt.

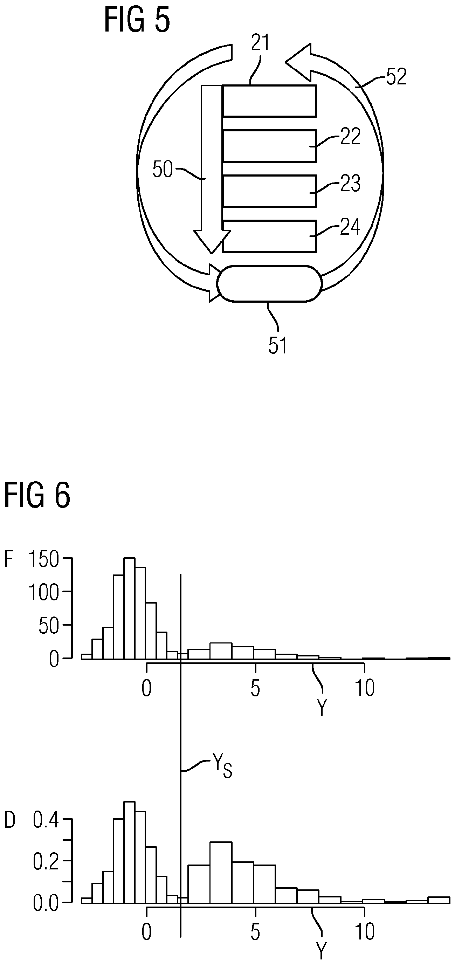

FIG 5 zeigt welche Schritte der aus FIG 4 bekannten Schritte nachvollzogen werden sollten.

Die Bearbeitungsabfolge 50 mit den Schritten Resampling 21, Training 22, Vorhersage 23 und der Qualitätskontrolle 24 kann bei Verwendung von Personalcomputern einige hunderte Male wiederholt werden, auch tausende Male, typischer Weise genau tausendmal.

Können die gefundenen Analyseparameter, insbesondere die Signifikanzparameter $I_i$ nicht weiter optimiert werden, so bricht die innere Schleife mit der Bearbeitungsabfolge 50 ab. Die innere Schleife kann bei jeder Abarbeitung eine Klassifizierungsregel vorschlagen. Entsprechendes gilt bei der Ausführung der äußeren Schleife 52.

Sodann findet eine Abfrage 51 statt, ob ein Signifikanzparameter $I_i$ derart klein ist, dass er keine verwertbare Information zum Klassifikationsindex beitragen kann. Wird ein derartiger Signifikanzparameter $I_i$ gefunden, so werden sämtliche Analyseparameter, die zum jeweiligen optischen Messwert gehören nicht weiter berücksichtigt, dazu gehören die Standardabweichung $\sigma_i$, der Mittelwert $E_i$ und der Signifikanzparameter $I_i$ des irrelevanten, optischen Merkmals.

Wurde beispielsweise erkannt, dass die Anzahl von Fragmenten in der Dispersion für die Malariadiagnose unerheblich ist, so hat der Signifikanzparameter, der beispielsweise $I_{28}$ sein könnte einen Wert angenommen, der sehr nah bei null liegt. Somit wird nachfolgend die Anzahl von Fragmenten nicht mehr zur Malariadiagnose herangezogen, indem die Standardabweichung $\sigma_{28}$, der Mittelwert $E_{28}$ und der Signifikanzparameter $I_{28}$ nicht mehr berücksichtigt werden. Dies kann in einem Computerprogramm dadurch erreicht werden, dass man $I_{28}$ auf null setzt ($I_{28} = 0$). Die Anzahl n wird um eins verringert, womit beispielsweise ausgehend von n = 29 nur noch n = 28 optische Merkmale berücksichtigt werden. Hierbei würde das optische Merkmal i = 29 die Zählernummer 28 erhalten, womit der erneute Start 52 der äußeren Schleife nunmehr mit 28 optischen Messwerten fortgesetzt werden kann.

FIG 6 zeigt zwei Diagramme, wobei das obere die Frequenz (Häufigkeit) von Klassifikationsindizes Y für eine negative Klassifikation (schmale Balken) und eine positive Klassifikation (breite Balken) über den Klassifikationsindex Y abgetragen sind. Aus dem oberen Diagramm ist ersichtlich, dass für die positive Klassifikation deutlich weniger Datensätze zur Verfügung standen, als für eine negative Klassifikation.

Des Weiteren wurde die Kenntnis der tatsächlich vorliegenden Klassifikation insoweit in das Diagramm einbezogen, dass bekannte positive Klassifikationen in der Verteilung mit breiten Balken gekennzeichnet sind und negative Klassifikationen in einer Verteilung mit schmalen Balken dargestellt sind.

Um vom oberen Diagramm zum unteren Diagramm zu gelangen, werden die beiden Verteilungen unterschiedlicher Klassifikationen als Wahrscheinlichkeitsdichteverteilung angesehen, und von daher entsprechend normiert. Diese Normierung kann beispielsweise den Flächeninhalt der jeweiligen Verteilungen auf eins setzen. Andere Normierungen sind gegebenenfalls auch sinnvoll. Die Normierungen stellen sicher, dass die unterschiedliche Zahl an Klassifikationen in den Eichdaten keinen Einfluss auf die zu bestimmende Klassifikationsregel nimmt.

Idealerweise würde man sich eine deutliche Separierung zwischen den gezeigten Wahrscheinlichkeitsdichteverteilungen D wünschen. Auf diese Weise wäre eine Schwelle $Y_s$ sehr leicht bestimmbar, indem nämlich $Y_s$ zwischen

den Wertebereichen der beiden Verteilungen festgelegt würde. In vorliegendem Fall der Malariadiagnose, verhält es sich so, dass für einen Bereich um die festgelegte Schwelle $Y_s$ die Wahrscheinlichkeit einer falschen Klassifikation möglich ist, weil der Klassifikationsindex Y in diesem Bereich nur unzureichend Aussagekraft besitzt. Je näher der Klassifikationsindex Y an einer Testdispersion dem Schwellenwert $Y_s$ liegt, umso wahrscheinlicher ist eine unkorrekte Klassifikation.

Eine Diskriminanzanalyse der in FIG 6 vorliegenden Verteilungen führt zu einer Festlegung der Analyseparameter, wie sie in der nachfolgenden Tabelle für das Analyseverfahren zur Malariadiagnoseunterstützung angegeben ist. Die Anzahl der Summanden zur Bestimmung des Klassifikationsindex beträgt 29 und ist identisch zu der Anzahl der berücksichtigten optischen Merkmale, die beim Test der Testdispersion tatsächlich eine Rolle spielen. In der Tat wurde mit 500 optischen Merkmalen begonnen, wobei 471 davon im Laufe der Anwendung des Ermittlungsverfahrens einen sehr kleinen Signifikanzparameter $l_i$ zu Tage brachten und aus dem Satz der zu berücksichtigenden Merkmale i entfernt wurden. Die Analyseparameter $l_i, E_i, \sigma_i$ der verbliebenen 29 optischen Merkmale i lauten:

Tabelle 1: Die vorstehende Tabelle listet die Analyseparameter $l_i, E_i, \sigma_i$ auf, wobei sowohl der zugehörige Messwert $P_i$, als auch die aufgelisteten Analyseparameter $l_i$, $E_i$, $\sigma_i$ dem jeweiligen optischen Merkmal i zugeordnet sind, und wobei der Parameter i als Nummer des jeweiligen optischen Merkmals zu verstehen ist.

| i | $l_i$ | $E_i$ | $\sigma_i$ |
|---|---|---|---|
| 1 | 0,0243143292327641 | 3,85878279704797 | 0,996350482855477 |
| 2 | 1,05744699221905 | 86,2085734326568 | 12,953846866348 |
| 3 | 1,3620780210087 | 31,3808859769373 | 2,65337438754902 |
| 4 | 0,298681341952128 | 16,7073148570111 | 3,05622820486547 |
| 5 | 0,039685909568696 | 3,18916825922509 | 0,610392795099183 |
| 6 | 0,802606052864478 | 22,4169387250923 | 6,67725822054997 |
| 7 | 0,013290099049454 | 1,80846317896679 | 0,490486418856065 |
| 8 | 0,172647099054505 | 0,602234248154981 | 0,216930342394397 |
| 9 | 0,0796067332070757 | 7,38290569464945 | 11,6539118822964 |
| 10 | 0,197381907473051 | 4,55739805811808 | 10,0180223771838 |
| 11 | 0,399165319578081 | 19,4079367287823 | 22,1428759582116 |
| 12 | 0,0494342161138479 | 1,24748103597786 | 2,31647351118736 |
| 13 | 1,44026210168956 | 26,9894740940959 | 5,0938073590873 |
| 14 | 0,520893299030793 | 4,28650641605166 | 1,07368357499902 |
| 15 | 0,284413335456635 | 16,4872902721402 | 12,5135939166568 |
| 16 | 0,531892835621228 | 7,23708487084871 | 8,95211789259242 |
| 17 | 0,698987246767777 | 336,37508399262 | 233,481906156752 |
| 18 | 0,488427163694765 | 9,19492226291513 | 2,00576799720274 |
| 19 | 0,683216666590792 | 53,4022117638376 | 11,7120398800053 |
| 20 | 2,39463588229883 | 23,307653449262 | 2,81445425488391 |
| 21 | 0,493291518472633 | 5,742889099631 | 0,731852460724153 |
| 22 | 0,199742256744743 | 1,95712033394834 | 0,25494636006376 |
| 23 | 1,76951528599063 | 0,839687189114391 | 0,161239146887579 |
| 24 | 0,0918169689792613 | 1,37246023431734 | 0,00515681120954237 |
| 25 | 2,99867547853134 | 34,8957316577491 | 5,81915230603462 |
| 26 | 2,46145277256995 | 17,5466706845018 | 3,58836148561811 |
| 27 | 0,0213275666681148 | 8,33807254059041 | 7,74938961818539 |
| 28 | 0,477404018065088 | 0,0352406872693727 | 0,136793871627217 |

(fortgesetzt)

| i | l_i | E_i | σ_i |
|---|---|---|---|
| 29 | 0272433630134861 | 0,016729979704797 | 0,0197033898754757 |

Tabelle 2: Bei ADVIA handelt es sich um ein hämatomisches Analysesystem mit automatisierter Messwertermittlung. Die Messwerte $P_i$ werden mit ihrem jeweiligen Kürzel, wie sie in sogenannten ADVIA Exportdateien verwendet werden, angegeben und in der letzten Spalte definiert.

| i | $P_i$ | ADVIA Parameter/ optisches Merkmal | Definition des Messwerts $P_i$ zum jeweiligen Merkmal i |
|---|---|---|---|
| 1 | $P_1$ | RBC | Zählung der roten Blutkörperchen aus dem RBC/PLT-Kanal |
| 2 | $P_2$ | MCV | Mittleres Zellvolumen der gezählten roten Blutkörperchen (RBC) |
| 3 | $P_3$ | CHCM | Mittlere Hämoglobinkonzentration (g/dL) der Zellen (aus RBC) |
| 4 | $P_4$ | RDW | Dichteverteilungsbreite der roten Blutkörperchen (aus RBC) |
| 5 | $P_5$ | HDW | Breite der Hämoglobindichteverteilung (g/dL) |
| 6 | $P_6$ | plat mode | PLT Mode |
| 7 | $P_7$ | mu fit | $\mu$-FIT |
| 8 | $P_8$ | sig fit | $\sigma$-FIT |
| 9 | $P_9$ | micro_pcnt | Anzahl der RBC Zellen in Prozent mit weniger als 60 Femtoliter |
| 10 | $P_{10}$ | macro_pcnt | Anzahl der RBC Zellen in Prozent mit mehr als 120 Femtoliter |
| 11 | $P_{11}$ | hypo_pcnt | Anzahl der von roten Blutkörperchen in Prozent mit einer Hämoglobin Konzentration von weniger als 28 g/dL |
| 12 | $P_{12}$ | hyper_pcnt | Anzahl der von roten Blutkörperchen in Prozent mit einer Hämoglobin Konzentration von weniger als 41 g/dL |
| 13 | $P_{13}$ | H_mean | Mittelwert der Hämoglobinkonzentration in Pikogramm |
| 14 | $P_{14}$ | H_deviation | Breite der Hämoglobininhaltsverteilung in Pikogramm |
| 15 | $P_{15}$ | VHC_covar | Kovarianz der Hämoglobindichteverteilung |
| 16 | $P_{16}$ | MN_PMN_valley | Das BASO MN/PMN Minimum als Senke zwischen den MN und PMN Clustern des BASO Zytogramms |
| 17 | $P_{17}$ | PLT | Zählung der erkannten Blutplättchen |
| 18 | $P_{18}$ | MPV | Mittleres Blutplättchenvolumen |
| 19 | $P_{19}$ | PDW | Blutplättchenverteilungsbreite |
| 20 | $P_{20}$ | MPC | Mittlere Blutplättchen- Komponentenkonzentration |
| 21 | $P_{21}$ | PCDW | Verteilungsbreite der Blutplättchenkomponentenkonzentration |
| 22 | $P_{22}$ | MPM | Mittlere Blutplättchentrockenmasse |
| 23 | $P_{23}$ | PMDW | Verteilungsbreite der Blutplättchentrockenmasse |
| 24 | $P_{24}$ | PLT_Mean_n | Mittel eines Brechungsindex der Blutplättchen (aus PLT-Messung) |

(fortgesetzt)

| i | $P_i$ | ADVIA Parameter/ optisches Merkmal | Definition des Messwerts $P_i$ zum jeweiligen Merkmal i |
|---|---|---|---|
| 25 | $P_{25}$ | PLT_Mean_X | Mittelwert der Beugungsablenkung in X-Richtung |
| 26 | $P_{26}$ | PLT_Mean_Y | Mittelwert der Beugungsablenkung in Y-Richtung |
| 27 | $P_{27}$ | Large_PLT | große Blutplättchen (Anzahl) |
| 28 | $P_{28}$ | RBC_Fragments | Anzahl der gezählten Fragmente im PLT-Beugungszytogramm |
| 29 | $P_{29}$ | RBC_Ghosts | Anzahl der Ereignisse im PLT-Beugungszytogramm ohne Zuordnung |

An dieser Stelle wird eine Referenz zur WO 00/58727 (PCT/US00/08512) hergestellt, deren gesamter Offenbarungsgehalt durch explizite Bezugnahme in dieses Schutzrecht zu integrieren ist, insbesondere soweit es die darin verwendeten Messwerte und optischen Merkmale des offenbarten hämatologischen Analysesystems betrifft.

Im Ausführungsbeispiel der Tabellen wird zur Malariadiagnoseunterstützung eine Schwelle $Y_s = 1,822$ vorgeschlagen. Somit kann für jeden ermittelten Klassifikationsindex einer Blutprobe ein Diagnosevorschlag bei einem kleinen Restrisiko gegeben werden, nämlich Bei Y > 1,822 ein positiver Malariabefund und bei Y < 1,822 ein negativer Malariabefund. Y = 1,822 liefert keine Unterstützung.

Während der Qualitätskontrolle 24 der Malariadiagnoseunterstützung wurden die Spezifitätsverteilung 41 und die Sensitivitätsverteilung 40 ermittelt und in einem zweidimensionalen Graphen gegeneinander abgetragen. Teilt man die Verteilungen in 4 gleiche Teile, so können die beiden mittleren Viertel für diese Betrachtung berücksichtigt werden. Mit anderen Worten, es werden das IQR 42 (InterQuartile Range) von der Verteilung 40 und das IQR 43 von der Verteilung 41 berücksichtigt. Um beste Ergebnisse zu erzielen wird empfohlen, dass man Klassifikationsregeln auswählt, die innerhalb des gezeigten Schnittbereichs S angesiedelt sind, um höchsten medizinischen Anforderungen gerecht zu werden.

Bei einer einfachen Mittelwertbildung erreicht die Sensitivität 96,25 % und bei einer Betrachtung des IQR einen Bereich von 59 % bis 97,5 %. Die Spezifität erreicht im Mittel 98,29 % und bei einer Betrachtung des IQR einen Bereich von 97,98 % bis 89,60 %. Somit ist eine korrekte Klassifizierung höchstwahrscheinlich, wobei falsche Klassifizierungsvorschläge nur mit einem kleinen Restrisiko ausgeschlossen werden können.

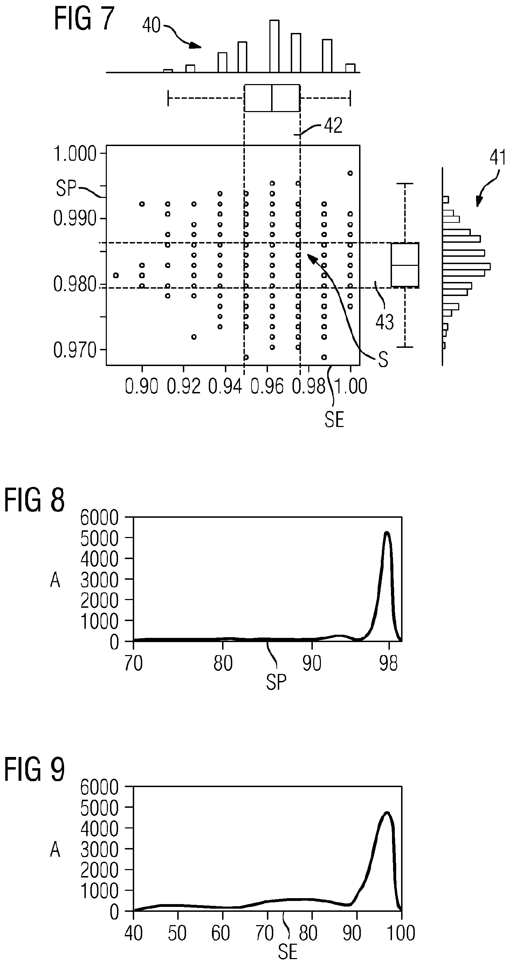

FIG 8 und FIG 9 zeigen jeweils die Anzahl festgestellter Spezifizitäten, beziehungsweise Sensitivitäten in Form von Histogrammen. Die Spezifität $S_P$ und die Sensitivität $S_E$ wurden bereits als Balkendiagramm in FIG 7 verwendet.

Zusammenfassend betrifft die Erfindung ein Analyseverfahren zur Klassifikationsunterstützung, ein Ermittlungsverfahren zur Bestimmung von Analyseparameter $Y_s$, $E_i$, $l_i$, $\sigma_i$ für das Analyseverfahren, ein Computerprogrammprodukt und ein optisches Analysesystem zur Klassifikationsunterstützung, bei dem basierend auf ersten und zweiten Eichdaten Analyseparameter $Y_s$, $E_i$, $l_i$, $\sigma_i$ festlegbar sind, die nach Regeln der Diskriminanzanalyse eine Klassifikationsunterstützung zur Verfügung stellen, die auf der Basis von Messwerten $P_i$ von optischen Merkmalen i, insbesondere organischer Dispersionen, deren Informationsgehalt zur Klassifikation, insbesondere Krankheitsdiagnose, einen Klassifikationsvorschlag oder Diagnosevorschlag im Vergleich mit einer Schwelle $Y_s$ erlauben.

**Patentansprüche**

1.  Analyseverfahren zur Klassifikationsunterstützung mit den Schritten

    - Ermittlung von Messwerten ($P_i$) zu optischen Merkmalen (i) einer zu klassifizierenden Testdispersion, wobei die Testdispersion aus einem Dispersionsmedium, insbesondere Blutplasma, und einer dispersen Phase gebildet ist und die disperse Phase Zellen oder Zellenbestandteile organischen Materials aufweist,
    - Berechnung eines Klassifikationsindex (Y), definiert durch

$$Y = \sum_{i=1}^{n} l_i * (P_i - E_i)/\sigma_i$$

wobei der Klassifikationsindex (Y) von einer Anzahl (n) der optischen Merkmale (i) der Testdispersion, einem Signifikanzparameter ($l_i$) des jeweiligen optischen Merkmals (i), einem Mittelwert ($E_i$) des jeweiligen optischen Merkmals (i) und einer Standardabweichung ($\sigma_i$) des jeweiligen optischen Merkmals (i) abhängt und

wobei der Mittelwert ($E_i$) des optischen Merkmals (i) und die Standardabweichung ($\sigma_i$) des optischen Merkmals (i) auf der Basis von ersten Eichdaten ermittelt wurden, wobei die ersten Eichdaten von Dispersionen mit einer negativen Klassifikation abgeleitet sind, insbesondere von den ersten Eichdaten und zweiten Eichdaten abgeleitet sind, wobei die zweiten Eichdaten von Dispersionen mit positiver Klassifikation abgeleitet sind und

wobei die Signifikanzparameter (li) von den ersten und von den zweiten Eichdaten mittels einer Diskriminanzanalyse abgeleitet sind, wobei bei der Diskriminanzanalyse insbesondere ein Bayes'sches Theorem verwendet wird und

wobei basierend auf einem ersten mittleren Klassifikationsindex (Y1), insbesondere einem ersten Schwerpunkt, der ersten Eichdaten und einem zweiten mittleren Klassifikationsindex (Y2), insbesondere einem zweiten Schwerpunkt, der zweiten Eichdaten ein Schwellenwert (YS) zur Klassifikationsunterstützung verwendet wird, wobei Y > YS positiver Klassifikation und Y < YS negativer Klassifikation zugeordnet ist und wobei eine positive Klassifikation ein Vorliegen eines Mangels, eines parasitären Befalls oder eines unnormalen Zustandes anzeigt, und wobei zur Berechnung des Klassifikationsindex (Y) wenigstens zwei optische Merkmale (i) berücksichtigt werden:

$$Y\ (n\ =\ 2)\ =\ l1{*}(P1\ -\ E1)/\sigma1\ +\ l2{*}(P2\ -\ E2)/\sigma2,$$

wobei n = 2 und ein erstes optisches Merkmal ein Maß für eine optische Streuung in eine transversale X-Richtung zu einem Analysestrahl (13) ist und ein zweites optisches Merkmal ein Maß für eine optische Streuung in einer zur X-Richtung senkrechten und zum Analysestrahl (13) senkrechten Y-Richtung ist, wobei der Analysestrahl (13) durch die Testdispersion (12) verläuft.

2. Analyseverfahren nach Anspruch 1, wobei der Mangel insbesondere eine Anämie, insbesondere eine Mittelmeer Anämie oder eine Sichelzellanämie, ist und wobei der parasitäre Befall insbesondere eine Leishmaniose oder eine andere parasitäre Infektion ist.

3. Analyseverfahren nach einem der Ansprüche 1 oder 2, ferner umfassend ein Ermittlungsverfahren zur Bestimmung von Analyseparametern ($Y_s$, $E_i$, $l_i$, $\sigma_i$) des Analyseverfahrens, wobei für eine festgelegte Anzahl (n) von optischen Merkmalen (i) die Analyseparameter ($Y_s$, $E_i$, $l_i$, $\sigma_i$) der Standardabweichung ($\sigma_i$), des Mittelwerts ($E_i$) und der Signifikanzparameter ($l_i$) basierend auf den ersten und den zweiten Eichdaten ermittelt werden.

4. Analyseverfahren nach Anspruch 3, wobei wenigstens ein erster Kontrollparameter, insbesondere ein Minimum oder ein Fitparameter, basierend auf den Analyseparametern ($Y_s$, $E_i$, $l_i$, $\sigma_i$) zur Bewertung der Klassifikationsunterstützung berechnet wird.

5. Analyseverfahren nach Anspruch 4, wobei wenigstens ein Teil der Analyseparameter ($Y_s$, $E_i$, $l_i$, $\sigma_i$) nach einer Bewertung durch den ersten Kontrollparameter angepasst wird, wobei insbesondere eine Anpassung des wenigstens einen Analyseparameters ($Y_s$, $E_i$, $l_i$, $\sigma_i$) und die Bewertung der Klassifikationsunterstützung, solange abwechselnd ausführbar sind, bis ein Verbesserung nicht mehr möglich oder nicht mehr erwünscht ist.

6. Analyseverfahren nach Anspruch 5, wobei der wenigstens eine Teil der Analyseparameter ($Y_s$, $E_i$, $l_i$, $\sigma_i$) die Signifikanzparameter ($l_i$) und/oder der Schwellenwert ($Y_s$) sind/ist.

7. Analyseverfahren nach Anspruch 5, wobei das Ermittlungsverfahren, sobald eine Verbesserung nicht mehr möglich oder nicht mehr erwünscht ist, mit einer reduzierten Anzahl von optischen Merkmalen (i) erneut ausführbar ist, wobei die Analyseparameter ($Y_s$, Ei, li, $\sigma$i) eines nicht signifikanten, optischen Merkmals (i) oder mehreren nicht signifikanten Merkmalen (i) nicht mehr berücksichtigt werden, wobei eine fehlende Signifikanz eines optischen Merkmals (i) anhand des Signifikanzparameters ($l_i$) festgestellt wird.

8. Analyseverfahren nach Anspruch 7, wobei fehlende Signifikanz vorliegt, wenn der Einfluss des optischen Merkmals (i) auf den Klassifikationsindex (Y) gering ist.

9. Computerprogrammprodukt, welches Instruktionen umfasst, welche beim Ausführen des Programms auf einem Computer, diesen Computer zur Ausführung der Schritte des Analyseverfahrens nach Anspruch 1 veranlassen.

10. Optisches Analysesystem (10), insbesondere ein hämatologisches Analysesystem (10), mit einer Analyseeinrichtung die für die Durchführung eines Analyseverfahrens nach Anspruch 1 konfiguriert ist.

**Claims**

1. Analysis method for classification assistance having the following steps:

   - ascertaining measured values ($P_i$) for optical features (i) of a test dispersion to be classified, wherein the test dispersion is formed from a dispersion medium, in particular blood plasma, and a dispersed phase and the dispersed phase has cells or cell components of organic materials,
   - calculating a classification index (Y), defined by

$$Y = \sum_{i=1}^{n} l_i * (P_i - E_i)/\sigma_i$$

   wherein the classification index (Y) is dependent on a number (n) of the optical features (i) of the test dispersion, a significance parameter ($l_i$) of the respective optical feature (i), a mean value ($E_i$) of the respective optical feature (i), and a standard deviation ($\sigma_i$) of the respective optical feature (i), and wherein the mean value ($E_i$) of the optical feature (i) and the standard deviation ($\sigma_i$) of the optical feature (i) were ascertained on the basis of first calibration data, wherein the first calibration data are derived from dispersions having a negative classification, in particular are derived from the first calibration data and second calibration data, wherein the second calibration data are derived from dispersions having positive classification, and wherein the significance parameters ($l_i$) are derived from the first and from the second calibration data by means of a discrimination analysis, wherein in particular a Bayesian theory is used in the discrimination analysis, and wherein, based on a first mean classification index ($Y_1$), in particular a first focal point, of the first calibration data and a second mean classification index ($Y_2$), in particular a second focal point, of the second calibration data, a threshold value ($Y_s$) is usable for the classification assistance, wherein $Y > Y_s$ is assigned to positive classification and $Y < Y_s$ is assigned to negative classification, and wherein a positive classification indicates a presence of a deficiency, a parasitic infestation, or a nonnormal state, wherein at least two optical features (i) are taken into consideration for the calculation of the classification index (Y):

$$Y\,(n = 2) = l_1 * (P_1 - E_1)/\sigma_1 + l_2 * (P_2 - E_2)/\sigma_2,$$

   wherein n = 2 and a first optical feature is a measure of optical scattering in a transverse X direction in relation to an analysis beam (13) and a second optical feature is a measure of optical scattering in a Y direction, which is perpendicular to the X direction and also perpendicular to the analysis beam (13), wherein the analysis beam (13) extends through the test dispersion (12).

2. Analysis method according to Claim 1, wherein the deficiency is in particular an anemia, in particular a Mediterranean anemia or a sickle-cell anemia, and wherein the parasitic infestation is in particular a leishmaniasis or another parasitic infection.

3. Analysis method according to either of Claims 1 and 2, furthermore comprising an ascertainment method for determining analysis parameters ($Y_s$, $E_i$, $l_i$, $\sigma_i$) of the analysis method, wherein for an established number (n) of optical features (i), the analysis parameters ($Y_s$, $E_i$, $l_i$, $\sigma_i$) of the standard deviation ($\sigma_i$), the mean value ($E_i$), and the significance parameters ($l_i$) are ascertained based on the first and the second calibration data.

4. Analysis method according to Claim 3, wherein at least one first control parameter, in particular a minimum or a fit parameter, is calculated based on the analysis parameters ($Y_s$, $E_i$, $l_i$, $\sigma_i$) to evaluate the classification assistance.

5. Analysis method according to Claim 4, wherein at least a part of the analysis parameters ($Y_s$, $E_i$, $l_i$, $\sigma_i$) is adapted after an evaluation by the first control parameter, wherein in particular an adaptation of the at least one analysis

parameter ($Y_s$, $E_i$, $l_i$, $\sigma_i$) and the evaluation of the classification assistance are executable alternately until an improvement is no longer possible or is no longer desirable.

6.  Analysis method according to Claim 5, wherein the at least one part of the analysis parameters ($Y_s$, $E_i$, $l_i$, $\sigma_i$) are/is the significance parameters ($l_i$) and/or the threshold value ($Y_s$) .

7.  Analysis method according to Claim 5, wherein the ascertainment method, as soon as an improvement is no longer possible or is no longer desired, is executable again using a reduced number of optical features (i), wherein the analysis parameters ($Y_s$, $E_i$, $l_i$, $\sigma_i$) of one nonsignificant optical feature (i) or multiple nonsignificant features (i) are no longer taken into consideration, wherein a lack of significance of an optical feature (i) is determined on the basis of the significance parameter ($l_i$).

8.  Analysis method according to Claim 7 wherein a lack of significance exists if the influence of the optical feature (i) on the classification index (Y) is small.

9.  Computer program product, which comprises instructions which, during the execution of the program on a computer, cause said computer to execute the steps of the analysis method according to Claim 1.

10. Optical analysis system (10), in particular a hematological analysis system (10), having an analysis unit configured for carrying out an analysis method according to Claim 1.

**Revendications**

1.  Procédé d'analyse au soutien d'une classification, comprenant les stades :

    - détermination de valeurs ($P_i$) de mesure de caractéristiques (i) optiques d'une dispersion de test à classer, la dispersion de test étant formée d'un milieu de dispersion, notamment de plasma sanguin, et d'une phase dispersée et la phase dispersée ayant des cellules ou des constituants de cellules de matières organiques,
    - calcul d'un indice (Y) de classification, défini par

    $$y = \sum_{i=l}^{n} l_i * (P_i - E_i)/\sigma_i$$

    dans laquelle l'indice (Y) de classification dépend d'un nombre (n) des caractéristiques (Y) optiques de la dispersion de test, d'un paramètre ($l_i$) de signification de la caractéristique (i) optique respective d'une valeur ($E_i$) moyenne de la caractéristique (i) optique respective et d'un écart-type ($\sigma_i$) de la caractéristique (i) optique respective et
    dans lequel on a déterminé la valeur ($E_i$) moyenne de la caractéristique (i) optique et l'écart-type ($\sigma_i$) optique sur la base de premières données d'étalonnage, les premières données d'étalonnage étant déduites de dispersions avec une classification négative, en étant déduites notamment de premières données d'étalonnage et de deuxièmes données d'étalonnage, les deuxièmes données d'étalonnage étant déduites de dispersions avec une classification positive et
    les paramètres ($l_i$) de signification sont déduits des premières et des deuxièmes données d'étalonnage au moyen d'une analyse discriminante, dans lequel, dans l'analyse discriminante, on utilise notamment un théorème de Bayes et
    dans lequel, sur la base d'un premier indice (Y1) moyen de classification, notamment d'un premier centre de gravité, des premières données d'étalonnage et d'un deuxième indice (Y2) de classification, notamment d'un deuxième centre de gravité, des deuxièmes données d'étalonnage, on utilise une valeur (YS) de seuil au soutien d'une classification, dans lequel Y < YS est associé à une classification positive et Y > YS est associé à une classification négative et
    dans lequel une classification positive indique la présence d'une carence, d'une attaque parasitaire ou d'un état anormal et dans lequel, pour le calcul de l'indice (Y) de classification, on prend en compte au moins deux caractéristiques (Y) optiques :

$$Y\,(n\,=\,2)\,=\,l1{*}(P1\,-\,E1)/\sigma1\,+\,l2{*}(P2\,-\,E2)/\sigma2,$$

dans laquelle n = 2 et une première caractéristique optique est une valeur d'une dispersion optique dans une direction X transversale au faisceau (13) d'analyse et une deuxième caractéristique optique est une valeur d'une dispersion optique dans une direction Y perpendiculaire à la direction X et perpendiculaire au faisceau (13) d'analyse, le faisceau (13) d'analyse passant dans la dispersion (12) de test.

2. Procédé d'analyse suivant la revendication 1, dans lequel la carence est notamment une anémie, notamment une anémie méditerranéenne ou une anémie à hématie falciforme, et dans lequel l'attaque parasitaire est notamment une leishmaniose ou une autre infection parasitaire.

3. Procédé d'analyse suivant l'une des revendications 1 ou 2, comprenant, en outre, un procédé de détermination pour déterminer des paramètres ($Y_s$, $E_i$, $l_i$, $\sigma_i$) d'analyse du procédé d'analyse, dans lequel, pour un nombre (n) fixé de caractéristiques (i) optiques, on détermine les paramètres ($Y_s$, $E_i$, $l_i$, $\sigma_i$) d'analyse de l'écart-type ($\sigma_i$), de la valeur moyenne ($E_i$) et des paramètres ($l_i$) de signification sur la base des premières et des deuxième données d'étalonnage.

4. Procédé d'analyse suivant la revendication 3, dans lequel on calcule, pour l'évaluation du soutien à la classification, au moins un premier paramètre de contrôle, notamment un minimum, ou un paramètre d'ajustement sur la base des paramètres ($Y_s$, $E_i$, $l_i$, $\sigma_i$) d'analyse.

5. Procédé d'analyse suivant la revendication 4, dans lequel on adapte au moins une partie des paramètres ($Y_s$, $E_i$, $l_i$, $\sigma_i$) d'analyse, après une évaluation par le premier paramètre de contrôle, dans lequel une adaptation du au moins un paramètre ($Y_s$, $E_i$, $l_i$, $\sigma_i$) d'analyse et l'évaluation du soutien à la classification peuvent être réalisées en alternance, jusqu'à ce qu'une amélioration ne soit plus possible ou ne soit plus souhaitée.

6. Procédé d'analyse suivant la revendication 5, dans lequel la au moins une partie des paramètres ($Y_s$, $E_i$, $l_i$, $\sigma_i$) d'analyse est le paramètre ($l_i$) de signification et/ou la valeur ($Y_s$) de seuil.

7. Procédé d'analyse suivant la revendication 5, dans lequel le procédé de détermination est, dès qu'une amélioration n'est plus possible ou n'est plus souhaitée, effectué à nouveau avec un nombre réduit de caractéristiques (i) optiques, dans lequel on ne tient plus compte des paramètres ($Y_s$, $E_i$, $l_i$, $\sigma_i$) d'analyse d'une caractéristique (i) optique non-significative ou de plusieurs caractéristiques (i) non-significatives, dans lequel on constate un manque de signification d'une caractéristique (i) optique à l'aide du paramètre ($l_i$) de signification.

8. Procédé d'analyse suivant la revendication 7, dans lequel il y a un manque de signification, lorsque l'influence de la caractéristique (i) optique sur l'indice (Y) de classification est petit.

9. Produit de programme d'ordinateur, qui comprend des instructions, qui font que, lorsque le programme est réalisé sur un ordinateur, cet ordinateur exécute les stades du procédé d'analyse suivant la revendication 1.

10. Système (10) d'analyse optique, notamment système (10) d'analyse hématologique, comprenant un dispositif d'analyse configuré pour effectuer un procédé d'analyse suivant la revendication 1.

## FIG 1

## FIG 2

## FIG 3

## FIG 4

# FIG 5

# FIG 6

FIG 7

FIG 8

FIG 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2011178716 A1
- US 2010273168 A1
- EP 2280278 A1
- WO 2013093913 A1
- WO 0058727 A
- US 0008512 W